# EUROPEAN PATENT APPLICATION

(11) **EP 4 649 971 A1**
(43) Date of publication of application: **19.11.2025**
(21) Application number: 25173172.5
(22) Date of filing: 29.04.2025
(51) Int. Cl.: A61M 1/16, A61M 1/36

(54) **BLOOD PURIFICATION APPARATUS**

(30) Priority: 17.05.2024 JP 2024080605
(71) Applicant: Shibuya Corporation, Kanazawa-shi, Ishikawa 920-8681 (JP)
(72) Inventor: MATSUZAKI, Kosho, Ishikawa, 920-8681 (JP); FUJII, Masayuki, Ishikawa, 920-8681 (JP); TAKIGAMI, Shohei, Ishikawa, 920-8681 (JP)
(74) Representative: Mewburn Ellis LLP

(57) **Abstract**

An object is to perform a procedure to drain fluid from a fluid replenishment passage. A fluid replenishment circuit 11 positioned between a dialysis fluid supply passage 4A and a blood circuit 3 is provided with an online port 13 to and from which a fluid replenishment passage 11a can be attached and detached. The online port 13 is provided with a first bypass passage 42 and an open/close valve (a fifteenth open/close valve V15) that allow a space formed between an inner port and an outer port to communicate with the dialysis fluid supply passage 4A.

In a fluid draining procedure, the fifteenth open/close valve V15 on the first bypass passage 42 is opened, and fluid in a dialysis fluid retrieval passage 4B is discharged by a water removal pump 38 on a water removal passage 37. As a result, air flows into the first bypass passage 42 through the outer port, and the air is further caused to flow via the dialysis fluid supply passage 4A through a replenishment fluid supply passage 11b into the blood circuit 3, so that fluid is removed from the fluid replenishment passage 11a.

## Description

### BACKGROUND OF THE INVENTION

### Field of the Invention

The present invention relates to a blood purification apparatus, and more specifically to a blood purification apparatus that makes it possible to efficiently eliminate fluid remaining in a blood circuit after a blood returning procedure.

### Description of the Related Art

There has been known a blood purification apparatus, including: a blood circuit including an artery passage and a vein passage connected to a blood purifier; and a dialysis fluid circuit including a dialysis fluid supply passage and a dialysis fluid retrieval passage connected to the blood purifier (Japanese Patent No. 4257602 and Japanese Laid-Open Patent Application No. 2021-145723).

For the blood purification apparatus structured as described above, a blood returning procedure is performed after dialysis treatment to return blood remaining in the blood purifier and the blood circuit to the patient. In the blood returning procedure, a fluid such as physiological saline or a dialysis fluid is generally used.

Further, after the blood returning procedure, the fluid remains in the blood circuit and a dialyzer. Thus, disposal in this state would cause the possibility of a contamination accident from the remaining fluid being scattered. In addition, there has been a problem of extra costs for processing the waste because the weight of the waste would be heavier by the weight of the remaining fluid.

To cope with the above, the blood purification apparatus disclosed in Japanese Patent No. 4257602 is configured to remove the remaining fluid in the blood circuit and the dialyzer, by opening a fluid disposal valve on the dialysis fluid retrieval passage while a communication opening of the dialyzer positioned on the dialysis fluid supply passage side is allowed to communicate with ambient air and further operating a blood pump provided for the blood circuit so as to cause air to flow in through the dialysis fluid supply passage.

In contrast, the blood purification apparatus disclosed in Japanese Laid-Open Patent Application No. 2021-145723 includes, for the purpose of fluid replenishment at the time of dialysis treatment, fluid replenishment passages provided branching off from the blood circuit. Among those, the fluid replenishment passage connected to the blood circuit side is connected to an online port provided for a dialysis apparatus.

In this connection, in the blood purification apparatus disclosed in Japanese Laid-Open Patent Application No. 2021-145723, the fluid replenishment passages are provided with a fluid replenishment pump. Thus, by operating the fluid replenishment pump, it is possible to remove fluid from the fluid replenishment passages after the blood returning procedure.

However, there have been known some blood purification apparatuses that include no fluid replenishment pump on fluid replenishment passages. In such configurations, it is not possible to remove fluid from the fluid replenishment passages by simply allowing a communication opening of the dialyzer positioned on the dialysis fluid supply passage side to communicate with the ambient air, as described in Japanese Patent No. 4257602. Thus, the problem is that fluid draining procedures can be cumbersome.

In view of the problems described above, the present invention provides a blood purification apparatus that makes it possible to efficiently perform a procedure to drain fluid also from a fluid replenishment passage.

### SUMMARY OF THE INVENTION

More specifically, the blood purification apparatus set forth in claim 1 is a blood purification apparatus that includes a blood purifier, an interior of which is divided into a blood flow path and a dialysis fluid flow path by a blood purification membrane, a blood circuit having an artery passage and a vein passage connected to the blood flow path of the blood purifier, a dialysis fluid circuit having a dialysis fluid supply passage and a dialysis fluid retrieval passage connected to the dialysis fluid flow path of the blood purifier, a blood pump provided for the artery passage, a replenishment fluid supply passage connected to the dialysis fluid supply passage, a fluid replenishment passage connected to the blood circuit, an online port which is coupled with the replenishment fluid supply passage and to and from which the fluid replenishment passage can be attached and detached, a water removal passage connected to the dialysis fluid retrieval passage, and a water removal pump provided for the water removal passage, the blood purification apparatus being characterized in that:
the online port includes an inner port connecting the replenishment fluid supply passage to the fluid replenishment passage and an outer port having a tubular shape and being provided around the inner port, and
further provided are a first bypass passage allowing a space formed between the inner port and the outer port to communicate with the dialysis fluid supply passage; and an open/close valve configured to open and close the first bypass passage.

In a fluid draining procedure to eliminate fluid remaining in the blood circuit, by opening the open/close valve on the first bypass passage and discharging fluid from the dialysis fluid retrieval passage by using the water removal pump, air is caused to flow into the first bypass passage through the outer port, and the air is further caused to flow via the dialysis fluid supply passage through the replenishment fluid supply passage toward the blood circuit, so as to thereby remove fluid from the fluid replenishment passage.

### Advantageous Effects of Invention

According to the invention set forth in claim 1, even when the blood purification apparatus is provided with no fluid replenishment pump for the fluid replenishment passage, by causing air to flow through the fluid replenishment passage via the outer port provided for the online port at the time of the fluid draining procedure, it is possible to use the air to eliminate the fluid from the fluid replenishment passage provided on the blood circuit side.

### BRIEF DESCRIPTION OF THE DRAWINGS

FIG. 1 is a circuit diagram of a dialysis apparatus according to an embodiment of the present disclosure;
FIG. 2 is a cross-sectional view of an online port in a closed state;
FIG. 3 is a cross-sectional view of the online port in a connected state;
FIG. 4 is a diagram for explaining steps in a fluid draining procedure for a fluid replenishment passage;
FIG. 5 is a diagram for explaining steps in a fluid draining procedure for a priming passage; and
FIG. 6 is a diagram for explaining steps in a fluid draining procedure for a blood circuit.

### DETAILED DESCRIPTION OF THE INVENTION

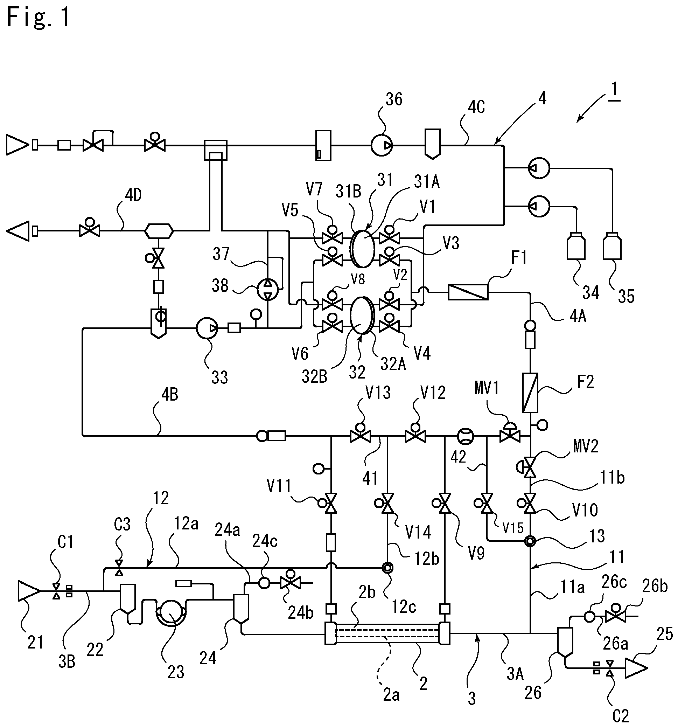

The following will describe the present invention with regard to the embodiment shown in the drawings. FIG. 1 shows a blood purification apparatus 1 that performs hemodialysis being blood purification treatment and that includes: a dialyzer 2 serving as a blood purifier; a blood circuit 3 connected to the dialyzer 2 and configured to cause blood to flow; and a dialysis fluid circuit 4 connected to the dialyzer 2 and configured to cause a dialysis fluid to flow. Further, the blood purification apparatus 1 is configured to be controlled by controlling device (not shown).

At the time of performing dialysis treatment, a new dialyzer 2 and a new blood circuit 3 are attached to the blood purification apparatus 1. Prior to the dialysis treatment, a priming procedure is performed by filling those attached elements with a priming fluid.

Further, after the dialysis treatment, it is necessary to perform a blood returning procedure by which the blood that has flowed through the dialyzer 2 and the blood circuit 3 during the dialysis treatment is returned to the patient. In the blood returning procedure, a dialysis fluid is supplied to the dialyzer 2 and to the blood circuit 3 from the dialysis fluid circuit 4, so that the blood in the dialyzer 2 and the blood circuit 3 is pushed out to the patient by the dialysis fluid.

Further, the dialyzer 2 and the blood circuit 3 that have been used need to be disposed of as medical waste. On such occasion, if fluid remains in the dialyzer 2 and/or the blood circuit 3, there would be a possibility of a contamination accident caused by the remaining fluid being scattered. In addition, there has been a problem of extra costs for processing the waste because the weight of the waste would be heavier by the weight of the remaining fluid.

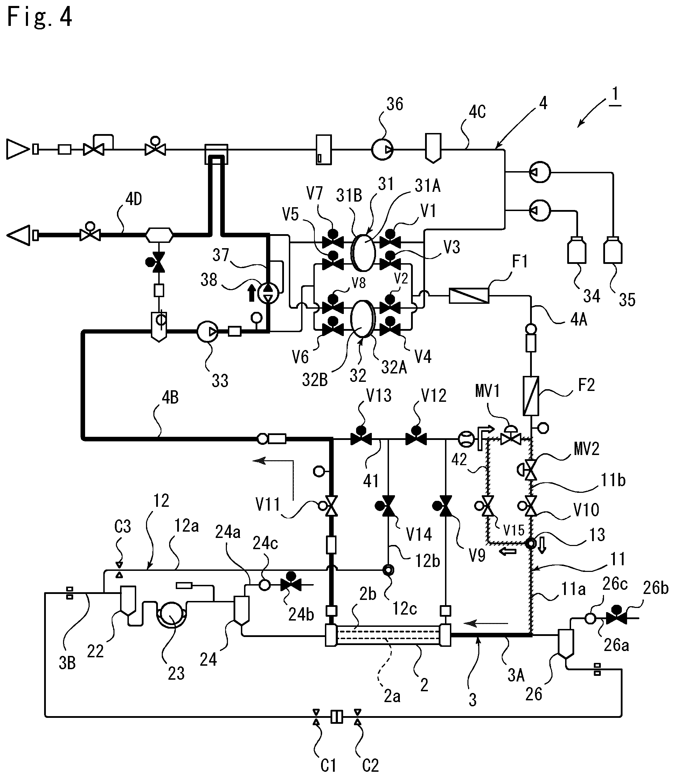

To cope with the circumstances described above, the blood purification apparatus 1 according to the present embodiment is configured so that, as shown in FIGS. 4 to 6, after the blood returning procedure, a fluid draining procedure is performed to eliminate the dialysis fluid remaining in the dialyzer 2 and the blood circuit 3.

The dialyzer 2 has a structure in which innumerable hollow fibers 2a serving as a blood purification membrane are accommodated inside a housing having a tubular shape. The inside of the hollow fibers 2a structures a blood flow path communicating with the blood circuit 3, whereas the outside thereof structures a dialysis fluid flow path communicating with the dialysis fluid circuit 4.

At the time of dialysis treatment, blood purification is carried out between the blood flowing through the blood flow path and the dialysis fluid flowing through the dialysis fluid flow path.

The blood circuit 3 includes: a vein passage 3A connected from the dialyzer 2 to a vein of the patient and used for returning the blood to the patient; and an artery passage 3B connected to an artery of the patient and used for supplying blood of the patient to the dialyzer 2.

Further, provided between the blood circuit 3 and the dialysis fluid circuit 4 of the present embodiment are a fluid replenishment circuit 11 used for replenishing fluid during the dialysis treatment and a priming passage 12 used primarily for the priming procedure.

One end of the artery passage 3B is connected to the dialyzer 2, whereas the other end is provided with a puncture needle 21 with which a blood vessel of the patient is punctured. In addition, provided between the dialyzer 2 and the puncture needle 21 are a first clamp C1, a drip chamber 22, a blood pump 23, and a drip chamber 24.

Among these elements, the drip chamber 24 is provided with an air escape passage 24a. The air escape passage 24a is provided with an air release valve 24b and an artery pressure sensor 24c.

One end of the vein passage 3A is connected to the dialyzer 2, whereas the other end is provided with a puncture needle 25 with which a blood vessel of the patient is punctured. In addition, provided between the dialyzer 2 and the puncture needle 25 are a drip chamber 26 and a second clamp C2.

Among these elements, the drip chamber 26 is provided with an air escape passage 26a. The air escape passage 26a is provided with an air release valve 26b and a vein pressure sensor 26c.

Further, at the time of performing the priming procedure or the fluid draining procedure, as shown in FIG. 4, it is possible to form a circulation path that is not externally open to the ambient air, by coupling a tip end part of the artery passage 3B with a tip end part of the vein passage 3A by using connector.

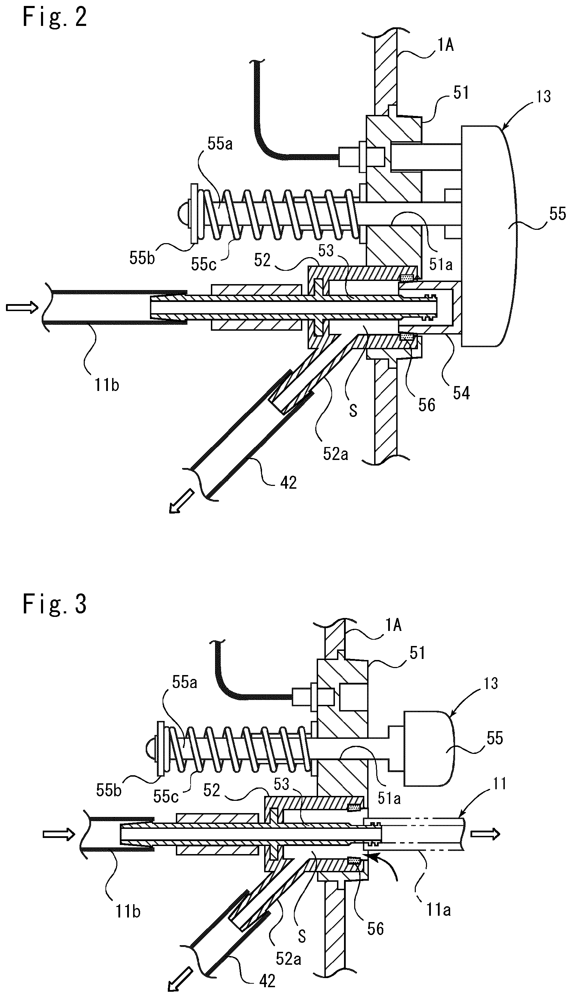

The fluid replenishment circuit 11 includes: a fluid replenishment passage 11a structuring the blood circuit 3 and being provided branching off from a certain location on the vein passage 3A that is positioned between the dialyzer 2 and the drip chamber 26; a replenishment fluid supply passage 11b structuring the dialysis fluid circuit 4; and an online port 13 being provided for a main body part 1A (see FIG. 2) of the blood purification apparatus 1 and connecting together the fluid replenishment passage 11a and the replenishment fluid supply passage 11b.

The fluid replenishment passage 11a positioned on the blood circuit side is configured to receive supply while being connected to the vein passage 3A. To perform the fluid draining procedure, it is necessary to remove the dialysis fluid from the fluid replenishment passage 11a, which is to be disposed of after being detached from the online port 13.

The priming passage 12 is provided branching off from a certain location on the artery passage 3B that is positioned between the first clamp C1 and the blood pump 23 and includes: a priming passage 12a positioned on the blood circuit side; a priming passage 12b positioned on the dialysis fluid circuit side; and a connection port 12c being provided for the main body part 1A and connecting together the priming passage 12a and the priming passage 12b.

As the connection port 12c, it is acceptable to use an element that is hitherto publicly known. The priming passage 12a positioned on the blood circuit side is provided with a third clamp C3.

The priming passage 12a positioned on the blood circuit side is configured to receive supply while being connected to the artery passage 3B. To perform the fluid draining procedure, it is necessary to remove the dialysis fluid from the priming passage 12a positioned on the blood circuit side, which is to be disposed of after being detached from the connection port 12c.

The dialysis fluid circuit 4 includes: a dialysis fluid supply passage 4A for supplying a fresh dialysis fluid to the dialyzer 2; a dialysis fluid retrieval passage 4B for retrieving used dialysis fluid that has passed through the dialyzer 2; a first dialysis fluid chamber 31 and a second dialysis fluid chamber 32 that are identical and store the dialysis fluid therein; and a fluid delivery pump 33 provided for the dialysis fluid retrieval passage 4B.

Formed on the inside of the first and the second dialysis fluid chambers 31 and 32 are supply compartments 31A and 32A storing fresh dialysis fluid therein and retrieval compartments 31B and 32B storing used dialysis fluid therein.

To each of the supply compartments 31A and 32A, the dialysis fluid supply passage 4A and a water supply passage 4C connected to purified water supply device (not shown) are each branched and connected. The branched passages of the water supply passage 4C are provided with fluid supply valves V1 and V2. The branched passages of the dialysis fluid supply passage 4A are provided with supply valves V3 and V4.

Meanwhile, to each of the retrieval compartments 31B and 32B, the dialysis fluid retrieval passage 4B and a fluid discharge passage 4D connected to a fluid discharge tank (not shown) are each branched and connected. The branched passages of the dialysis fluid retrieval passage 4B are provided with retrieval valves V5 and V6. The branched passages of the fluid discharge passage 4D are provided with fluid discharge valves V7 and V8.

Along the water supply passage 4C, a fluid A supply source 34 and a fluid B supply source 35 are connected for supplying undiluted fluid A and undiluted fluid B, which are undiluted fluids of the dialysis fluid, while a water supply pump 36 is provided on the upstream side thereof.

The dialysis fluid supply passage 4A is provided with a first dialysis fluid filter F1 and a second dialysis fluid filter F2 each realized with an endotoxin removal filter that purifies the dialysis fluid, as well as a first flow volume adjustment valve MV1 and a ninth open/close valve V9 controlled by the controlling device.

The replenishment fluid supply passage 11b structuring the fluid replenishment circuit 11 is connected to a certain location between the second dialysis fluid filter F2 and the first flow volume adjustment valve MV1. The replenishment fluid supply passage 11b is provided with a second flow volume adjustment valve MV2 and a tenth open/close valve V10 controlled by the controlling device.

As being controlled by the controlling device, the first flow volume adjustment valve MV1 on the dialysis fluid supply passage 4A and the second flow volume adjustment valve MV2 on the replenishment fluid supply passage 11b are capable of adjusting flow volumes of the dialysis fluid caused to flow into the dialysis fluid supply passage 4A and the fluid replenishment circuit 11.

The dialysis fluid retrieval passage 4B is provided with an eleventh open/close valve V11 controlled by the controlling device and the fluid delivery pump 33. Provided in a position adjacent to the fluid delivery pump 33 on the downstream side thereof is a water removal passage 37 positioned between the fluid delivery pump 33 and the fluid discharge passage 4D. The water removal passage 37 is provided with a water removal pump 38.

The water removal pump 38 is structured by using a cylinder pump and is capable of accurately controlling a flow volume to be delivered, under control of the controlling device.

A second bypass passage 41 according to the present invention is provided between the dialysis fluid supply passage 4A and the dialysis fluid retrieval passage 4B. The second bypass passage 41 is provided with a twelfth open/close valve V12 and a thirteenth open/close valve V13 controlled by the controlling device.

One end of the second bypass passage 41 is connected to a certain point on the dialysis fluid supply passage 4A that is positioned between the ninth open/close valve V9 and the first flow volume adjustment valve MV1. The other end is connected to a certain point on the dialysis fluid retrieval passage 4B that is positioned between the eleventh open/close valve V11 and the fluid delivery pump 33.

The priming passage 12b positioned on the dialysis fluid circuit side and structuring the priming passage 12 is connected to a certain location on the second bypass passage 41 that is between the twelfth open/close valve V12 and the thirteenth open/close valve V13. The priming passage 12b is provided with a fourteenth open/close valve V14 controlled by the controlling device.

With this arrangement, when the twelfth open/close valve V12 on the second bypass passage 41 is opened, while the thirteenth open/close valve V13 is closed, the blood circuit 3 communicates with the dialysis fluid supply passage 4A via the priming passage 12. On the contrary, when the twelfth open/close valve V12 is closed, while the thirteenth open/close valve V13 is opened, the blood circuit 3 communicates with the dialysis fluid retrieval passage 4B via the priming passage 12.

FIG. 2 and FIG. 3 show the online port 13 provided for the fluid replenishment circuit 11. FIG. 2 shows a closed state in which the online port 13 is closed by a lid member 54. FIG. 3 shows a connected state in which the fluid replenishment passage 11a is connected to the online port 13. In this situation, because the online port 13 of the present embodiment is hitherto publicly known from Japanese Laid-Open Patent Application No. 2021-145723 listed above, detailed explanations thereof will be omitted.

The online port 13 includes: a base member 51 fixed to the main body part 1A of the blood purification apparatus 1; an outer port 52 having the shape of a bottomed cylinder and being fixed to the base member 51; an inner port 53 having a tubular shape and being provided so as to penetrate the outer port 52; the lid member 54 configured to close the inner port 53 together with the outer port 52; and a lever 55 configured to move the lid member 54.

The base member 51 has a substantially disc shape and has a through hole 51a formed in a position adjacent to the outer port 52. A rod 55a provided for the lever 55 swingably penetrates the through hole 51a.

A certain part of the rod 55a protruding on the interior space side of the main body part 1A is provided with a flange 55b. By a spring 55c resiliently installed between the flange 55b and the base member 51, the lever 55 is constantly biased toward the main body part 1A side.

The outer port 52 has the shape of a bottomed cylinder. The opening part thereof is positioned on an end face of the base member 51, while the bottom part thereof is provided so as to protrude into the interior of the main body part 1A.

Further, in the vicinity of the opening part, a ring seal 56 is attached to the inner circumferential surface of the outer port 52. As a result of the ring seal 56 closely adhering to the outer circumferential surface of the lid member 54, the two members are held in a liquid-tight state.

A discharge opening 52a is formed in a certain part of the circular cylinder face of the outer port 52 that is positioned underneath. The discharge opening 52a is coupled with the first bypass passage 42 according to the present invention.

As shown in FIG. 1, the first bypass passage 42 is connected to a certain location on the dialysis fluid supply passage 4A that is between the first flow volume adjustment valve MV1 and the ninth open/close valve V9. Further, the first bypass passage 42 is provided with a fifteenth open/close valve V15 controlled by the controlling device.

The inner port 53 is structured by using a pipe-like member and penetrates the bottom face of the outer port 52, so that a space S is formed between the inner circumferential surface of the outer port 52 and the outer circumferential surface of the inner port 53.

Also, one end of the inner port 53 is provided so as to protrude to the outside of the main body part 1A. When the online port 13 is brought into the connected state, it is possible to attach the fluid replenishment passage 11a to a tip end part of the inner port 53.

Meanwhile, the other end part of the inner port 53 protrudes into the interior of the main body part 1A so as to be coupled with the replenishment fluid supply passage 11b. When the fluid replenishment passage 11a is connected to the inner port 53, the replenishment fluid supply passage 11b and the fluid replenishment passage 11a communicate with each other.

The lid member 54 is a member having the shape of a bottomed cylinder, while the bottom part thereof is coupled with and fixed to the lever 55. The outside diameter of the lid member 54 is substantially equal to the inside diameter of the outer port 52, while the inside diameter of the lid member 54 is larger than the diameter of the inner port 53.

With the configuration described above, in the closed state shown in FIG. 2, while the lid member 54 is inserted in the outer port 52, the outer circumferential surface of the lid member 54 closely adheres to the ring seal 56 provided on the inner circumferential surface of the outer port 52, so that the two members are held in the liquid-tight state.

Further, in the closed state, a gap is formed between the tip end part of the inner port 53 and the bottom part of the lid member 54, so that the space S formed between the inner port 53 and the outer port 52 is separated from the outside in a liquid-tight manner.

With the configuration described above, after the dialysis treatment is finished, when the online port 13 is brought into the closed state, while a cleaning fluid is supplied through the replenishment fluid supply passage 11b, the dialysis fluid is discharged through the tip end part of the inner port 53. In this manner, the tip end part of the inner port 53 exposed to the space S is cleaned. After that, the cleaning fluid is discharged through the discharge opening 52a via the first bypass passage 42 into the dialysis fluid supply passage 4A.

To change the online port 13 from the closed state shown in FIG. 2 into the opened state shown in FIG. 3, it is suggested that the lever 55 be pulled to the outside of the main body part 1A, so as to turn the lever 55 by approximately 90°, while the lid member 54 is positioned apart from the outer port 52.

As a result, the inner port 53 becomes exposed, so that it is possible to connect the fluid replenishment passage 11a to the tip end part of the inner port 53. Further, the dialysis fluid supplied through the replenishment fluid supply passage 11b flows through the inner port 53 and is subsequently supplied to the blood circuit 3 via the fluid replenishment passage 11a.

Further, when the online port 13 has been brought into the opened state, the space S formed between the inner port 53 and the outer port 52 is opened to the outside. Thus, as explained later, it is possible to cause ambient air to flow into the first bypass passage 42 via the discharge opening 52a.

Operations of the blood purification apparatus 1 structured as described above will be explained. To begin with, a flow of the dialysis fluid in the dialysis fluid circuit 4 at the time of the dialysis treatment will be explained.

To the supply compartment 31A of the first dialysis fluid chamber 31, water, the undiluted fluid A, and the undiluted fluid B are supplied from the purified water supply device, the fluid A supply source 34, and the fluid B supply source 35 via the water supply passage 4C, so as to be mixed together inside, and fresh dialysis fluid is thus prepared.

In this situation, when the water and the undiluted fluids have flowed into the supply compartment 31A, the capacity of the supply compartment 31A increases while a diaphragm is changing the shape thereof. In conjunction therewith, the capacity of the retrieval compartment 31B decreases, so that used dialysis fluid is discharged from the retrieval compartment 31B via the fluid discharge passage 4D.

Meanwhile, used dialysis fluid that has passed through the dialyzer 2 is supplied to the retrieval compartment 32B of the second dialysis fluid chamber 32 via the dialysis fluid retrieval passage 4B. As a result, the capacity of the retrieval compartment 32B increases. In conjunction therewith, the capacity of the supply compartment 32A decreases, so that fresh dialysis fluid is delivered from the supply compartment 32A to the dialyzer 2 via the dialysis fluid supply passage 4A.

After that, when the capacities of the retrieval compartment 31B of the first dialysis fluid chamber 31 and the supply compartment 32A of the second dialysis fluid chamber 32 have become zero, the open/closed states are switched between the fluid supply valves V1 and V2 and the fluid discharge valves V7 and V8, as well as the supply valves V3 and V4 and the retrieval valves V5 and V6 provided for the first and the second dialysis fluid chambers 31 and 32.

As a result, with respect to the first dialysis fluid chamber 31, the fresh dialysis fluid is supplied from the supply compartment 31A to the dialyzer 2, whereas the used dialysis fluid is retrieved into the retrieval compartment 31B. In contrast, with respect to the second dialysis fluid chamber 32, the fresh dialysis fluid is supplied to the supply compartment 32A, whereas the used dialysis fluid is discharged from the retrieval compartment 32B into the fluid discharge passage 4D.

After that, by repeatedly performing the above operations alternately, it is possible to continuously supply fresh dialysis fluid to the dialyzer 2 and to retrieve used dialysis fluid, by employing the dialysis fluid circuit 4. Thus, hemodialysis is carried out by the dialyzer 2 with the blood flowing through the blood circuit 3.

At the time of performing a fluid replenishment operation to replenish dialysis fluid for the patient during the dialysis treatment, the controlling device controls the first flow volume adjustment valve MV1 on the dialysis fluid supply passage 4A and the second flow volume adjustment valve MV2 on the replenishment fluid supply passage 11b and opens the tenth open/close valve V10 on the replenishment fluid supply passage 11b.

As a result, a part of the dialysis fluid flowing through the dialysis fluid supply passage 4A flows through the replenishment fluid supply passage 11b and subsequently flows into the fluid replenishment passage 11a via the online port 13, so as to further flow into the vein passage 3A. Thus, the fluid is replenished for the patient.

At the time of performing a water removal operation so as to remove water from blood of the patient during the dialysis treatment, the controlling device causes the water removal pump 38 provided for the water removal passage 37 to operate in a forward direction.

As a result, a part of the used dialysis fluid flowing through the dialysis fluid retrieval passage 4B is discharged into the fluid discharge passage 4D via the water removal passage 37. Consequently, a pressure difference occurs inside the dialyzer 2, so that excess water is removed from the blood.

Next, a priming procedure performed at the time of using the blood purification apparatus will be explained.

To perform the priming procedure, to begin with, an empty dialyzer 2 and an empty blood circuit 3 are attached to the blood purification apparatus 1. In addition, the fluid replenishment passage 11a structuring the fluid replenishment circuit 11 is connected to the online port 13, whereas the priming passage 12a structuring the priming passage 12 and being provided on the blood circuit side is connected to the connection port 12c.

In that state, when the dialysis fluid is caused flow through the dialysis fluid circuit 4 similarly to during the dialysis treatment, the dialysis fluid flows through the dialysis fluid supply passage 4A into the dialysis fluid flow path of the dialyzer 2, so as to be subsequently discharged into the dialysis fluid retrieval passage 4B, so that the dialysis fluid flow path is filled with the dialysis fluid.

Subsequently, similarly to during the fluid replenishment procedure, the first and the second flow volume adjustment valves MV1 and MV2 are controlled so as to cause the dialysis fluid in the dialysis fluid supply passage 4A to flow into the blood circuit 3 via the fluid replenishment circuit 11.

In that situation, as a result of bringing the blood pump 23 into operation while the artery passage 3B and the vein passage 3A of the blood circuit 3 are coupled with each other, the dialysis fluid that has flowed into the blood circuit 3 circulates in the blood circuit 3, so as to fill the blood flow path of the dialyzer 2 and the blood circuit 3.

Meanwhile, when the dialysis fluid keeps being supplied to the blood circuit 3, a certain amount of the dialysis fluid that has flowed in beyond the capacities of the dialyzer 2 and the blood circuit 3 is discharged through the priming passage 12. The discharged dialysis fluid is subsequently retrieved into the dialysis fluid retrieval passage 4B via the first bypass passage 42.

As a result of the priming operation described above, the fluid replenishment circuit 11 and the priming passage 12 connected to the blood circuit 3 are each filled with the dialysis fluid.

Next, a blood returning procedure will be explained in which the blood remaining in the blood circuit 3 after the dialysis treatment is returned to the patient.

During the blood returning procedure, the artery passage 3B and the vein passage 3A remain attached to the patient. In that state, similarly to during the fluid replenishment procedure, the first and the second flow volume adjustment valves MV1 and MV2 are controlled so as to cause the dialysis fluid in the dialysis fluid supply passage 4A to flow into the blood circuit 3 via the fluid replenishment circuit 11.

Further, the blood pump 23 for the blood circuit 3 is caused to rotate in the forward direction and the backward direction, so that the blood in the artery passage 3B and the vein passage 3A is pushed out toward the patient by the dialysis fluid that has been caused to flow into the blood circuit 3. As a result, the blood in the blood circuit 3 is returned to the patient.

Alternatively, at the time of the blood returning procedure, it is also acceptable to supply the dialysis fluid to the blood circuit 3 through the dialysis fluid supply passage 4A via the priming passage 12, so as to carry out the blood returning procedure.

As a result of the blood returning operation described above, the dialyzer 2 and the blood circuit 3 are filled with the dialysis fluid instead of blood. In this situation, the fluid replenishment circuit 11 and the priming passage 12 connected to the blood circuit 3 are also each filled with the dialysis fluid.

Next, a fluid draining procedure will be explained with reference to FIGS. 4 to 6, in which, after the blood returning procedure, the dialysis fluid is eliminated from the dialyzer 2 and the blood circuit 3.

As described earlier, when the blood returning procedure is completed, the dialyzer 2 and the blood circuit 3 are filled with the dialysis fluid. In that state, a medical worker detaches the puncture needles 21 and 25 from the patient and subsequently couples the tip end parts of the vein passage 3A and the artery passage 3B with each other by using coupling device (not shown) so that a circulation path is formed.

In that state, the medical worker instructs the controlling device to start the fluid draining procedure. The controlling device at first performs an operation to drain fluid from the fluid replenishment circuit 11 shown in FIG. 4.

In contrast to the abovementioned operation performed at the time of the dialysis treatment, the controlling device closes all of the fluid supply valves V1 and V2, the supply valves V3 and V4, the retrieval valves V5 and V6, and the fluid discharge valves V7 and V8 communicating with the first and the second dialysis fluid chambers 31 and 32.

Further, the controlling device closes the ninth open/close valve V9 on the dialysis fluid supply passage 4A, fully opens the first flow volume adjustment valve MV1 and the second flow volume adjustment valve MV2, and also opens the tenth open/close valve V10 on the replenishment fluid supply passage 11b and the fifteenth open/close valve V15 on the first bypass passage 42.

In addition, the controlling device brings the fluid delivery pump 33 on the dialysis fluid retrieval passage 4B and the water removal pump 38 on the water removal passage 37 into operation, so that the dialysis fluid in the dialysis fluid retrieval passage 4B is discharged into the fluid discharge passage 4D. Also, at this time, the blood pump 23 for the blood circuit 3 is stopped.

As a result, the dialysis fluid in the dialysis fluid retrieval passage 4B is delivered by the water removal pump 38 into the fluid discharge passage 4D, so that the dialysis fluid is discharged from the dialysis fluid flow path of the dialyzer 2. On the inside of the dialyzer 2, a pressure difference is caused between the dialysis fluid flow path and the blood flow path, so that the dialysis fluid moves from the blood flow path to the dialysis fluid flow path.

Meanwhile in the fluid replenishment circuit 11, because the fifteenth open/close valve V15 on the first bypass passage 42 is opened while the online port 13 is in the connected state as shown in FIG. 3, air is allowed to flow in through the discharge opening 52a of the outer port 52.

The air that has flowed in through the outer port 52 flows backward through the part shown with the hatching in FIG. 4, i.e., through the first bypass passage 42 into the dialysis fluid supply passage 4A and subsequently flows through the fluid replenishment circuit 11 into the vein passage 3A.

In this manner, the air pushes out the dialysis fluid that remained in the fluid replenishment circuit 11 into the vein passage 3A. In particular, the dialysis fluid is eliminated from the fluid replenishment passage 11a connected to the blood circuit 3.

In this situation, during the operation shown in FIG. 4, the controlling device has registered therein the capacity of the part through which the air flows, i.e., the capacity of the flow path structuring the first bypass passage 42, a part of the dialysis fluid supply passage 4A, and the fluid replenishment circuit 11. Thus, by controlling the water removal pump 38, the controlling device is configured to cause the dialysis fluid to be delivered in a first fluid delivery flow volume corresponding to the registered capacity.

When the dialysis fluid has been discharged through the dialysis fluid retrieval passage 4B into the fluid discharge passage 4D in the amount defined as the first fluid delivery flow volume, air flows in through the outer port 52 of the online port 13 in an amount equal to the first fluid delivery flow volume. Thus, it is possible to eliminate almost all of the dialysis fluid from the fluid replenishment circuit 11.

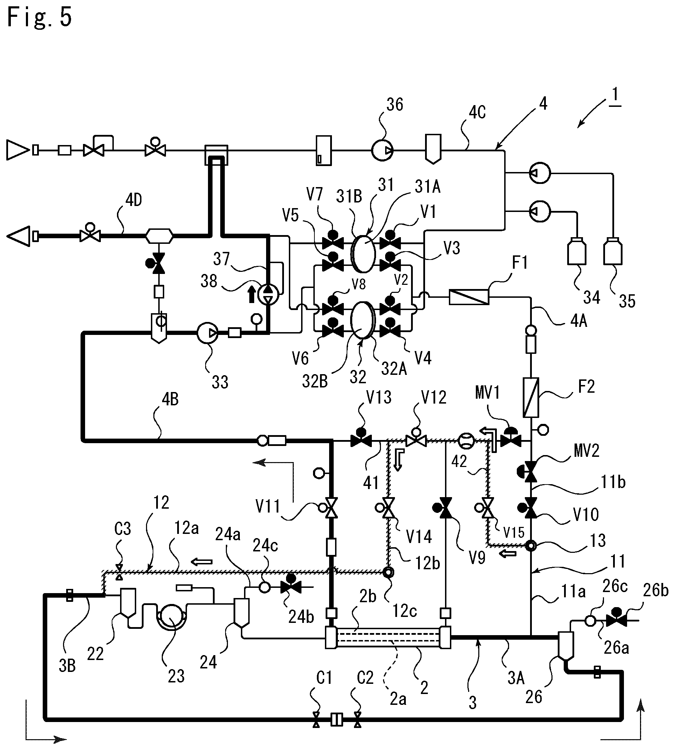

FIG. 5 shows an operation to drain fluid from the priming passage 12.

When the fluid draining procedure of the fluid replenishment circuit 11 shown in the state in FIG. 4 is completed, while keeping the fluid delivery pump 33 and the water removal pump 38 in operation, the controlling device closes the first and the second flow volume adjustment valves MV1 and MV2 and closes the tenth open/close valve V10 on the replenishment fluid supply passage 11b.

Further, the controlling device opens the twelfth open/close valve V12 on the second bypass passage 41 so as to allow the priming passage 12 to communicate with the dialysis fluid supply passage 4A and further opens the fourteenth open/close valve V14 and the third clamp C3 on the priming passage 12.

In FIG. 5 also, in the dialyzer 2, the dialysis fluid is flowing through the hollow fibers 2a from the blood flow path into the dialysis fluid flow path, as continued from the state shown in FIG. 4. In addition, at the online port 13, the inflow of the air through the outer port 52 is continued.

As a result, the air that has flowed in through the outer port 52 of the online port 13 flows through the first bypass passage 42 into the dialysis fluid supply passage 4A and subsequently flows through the second bypass passage 41 into the artery passage 3B via the priming passage 12.

The air mentioned above pushes out the dialysis fluid remaining in the priming passage 12 into the artery passage 3B. Thus, the dialysis fluid is eliminated from the priming passage 12.

In this situation also, the controlling device has registered therein, in advance, the capacity of the part through which the air flows, i.e., the capacity of the flow path structuring a part of the dialysis fluid supply passage 4A, a part of the second bypass passage 41, and the priming passage 12. Thus, by controlling the water removal pump 38, the controlling device is configured to cause the dialysis fluid to be delivered in a second fluid delivery flow volume corresponding to the registered capacity.

When the dialysis fluid has been discharged through the dialysis fluid retrieval passage 4B into the fluid discharge passage 4D in the amount defined as the second fluid delivery flow volume, air flows in through the outer port 52 of the online port 13 in an amount equal to the second fluid delivery flow volume. Thus, almost all of the dialysis fluid has been eliminated from the priming passage 12, and the fluid draining procedure for the priming passage 12a positioned on the blood circuit side is thus completed.

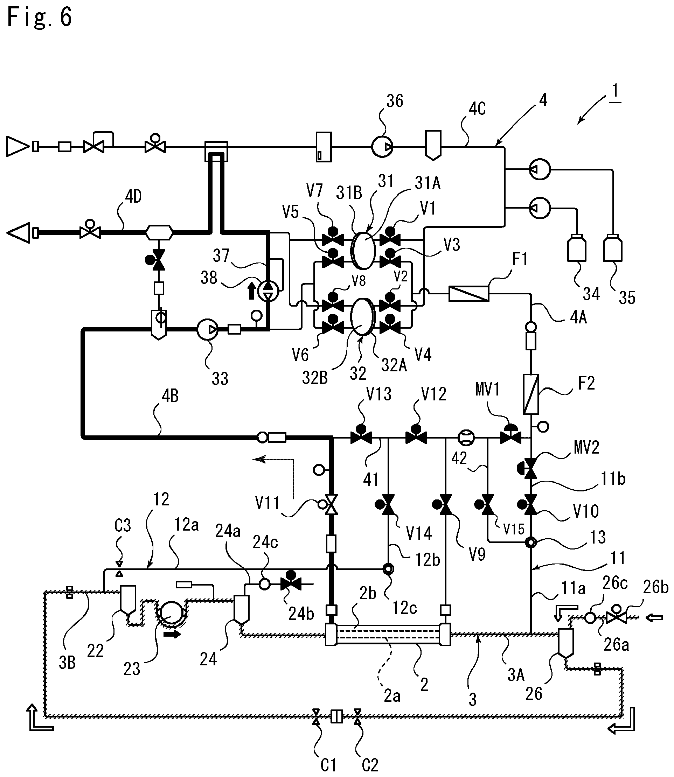

FIG. 6 shows an operation to drain fluid from the dialyzer 2 and the blood circuit 3.

In the state shown in FIG. 5, when the fluid draining procedure of the priming passage 12 is completed, while keeping the fluid delivery pump 33 and the water removal pump 38 in operation, the controlling device closes the fifteenth open/close valve V15 on the first bypass passage 42 connected to the online port 13, the twelfth open/close valve V12 on the second bypass passage 41, as well as the fourteenth open/close valve V14 and the third clamp C3 on the priming passage 12.

In addition, the controlling device opens the air release valve 26b provided for the air escape passage 26a of the drip chamber 26 on the vein passage 3A in the blood circuit 3 and further causes the blood pump 23 to operate in the forward direction.

In the dialyzer 2, as continued from the state shown in FIG. 5, the dialysis fluid is moving from the blood flow path to the dialysis fluid flow path, while the blood circuit 3 is forming a circulation path by having the vein passage 3A coupled with the artery passage 3B. Accordingly, the dialysis fluid delivered by the blood pump 23 moves, in the dialyzer 2, from the blood flow path to the dialysis fluid flow path and is subsequently discharged into the dialysis fluid retrieval passage 4B.

Meanwhile, the air that has flowed in through the air escape passage 26a of the drip chamber 26 flows from the drip chamber 26 into the vein passage 3A and subsequently pushes out the dialysis fluid remaining in the artery passage 3B toward the dialyzer 2 through the vein passage 3A.

By operating the blood pump 23 for a prescribed period of time, the controlling device causes, in the dialyzer 2, the dialysis fluid to be discharged into the dialysis fluid retrieval passage 4B and thus completes the fluid draining procedure of the dialyzer 2 and the blood circuit 3.

In this situation also, the controlling device has registered therein, in advance, the capacity of the flow path structuring the vein passage 3A and the artery passage 3B and the dialyzer 2. Thus, by controlling the water removal pump 38, the controlling device is configured to cause the dialysis fluid to be delivered in a third fluid delivery flow volume corresponding to the registered capacity.

When the dialysis fluid has been removed from the dialyzer 2 and the blood circuit 3 in this manner, a medical worker separates the fluid replenishment passage 11a from the online port 13 and separates the priming passage 12a provided on the blood circuit side from the connection port 12c. In addition, the dialyzer 2 and the blood circuit 3 are detached from the blood purification apparatus 1 and disposed of as medical waste.

As described above, the blood purification apparatus 1 according to the present embodiment includes the online port 13 shown in FIG. 2 and FIG. 3 and is thus capable of automatically performing, as shown in FIGS. 4 to 6, the fluid draining procedure on all the passages structuring the blood circuit 3 including the fluid replenishment passage 11a and the priming passage 12a.

More specifically, as for the fluid replenishment circuit 11, the ambient air is caused to flow into the first bypass passage 42 via the discharge opening 52a provided for the online port 13. Thus, by causing the air to flow through the dialysis fluid supply passage 4A into the fluid replenishment circuit 11, it is possible to eliminate the dialysis fluid from the fluid replenishment circuit 11.

Similarly, as for the priming passage 12 also, the ambient air is caused to flow into the first bypass passage 42 via the discharge opening 52a provided for the online port 13. Thus, by causing the air to flow through the dialysis fluid supply passage 4A into the second bypass passage 41 and the priming passage 12, it is possible to eliminate the dialysis fluid from the priming passage 12.

At the time of the fluid draining procedure, a medical worker simply needs to couple the artery passage 3B and the vein passage 3A with each other that have been detached from the patient and to perform necessary operations on the controlling device. Thus, it is possible to efficiently perform the series of operations involved in the fluid draining procedure.

Further, the operations shown in FIG. 4 and FIG. 5 may be performed in the reversed order. In addition, during the operation shown in FIG. 6, it is also acceptable to release other elements provided in the blood circuit 3 such as the air escape passage 24a of the drip chamber 24.

Further, the above embodiment was explained regarding a so-called personal dialysis apparatus configured to prepare the dialysis fluid in the first and the second dialysis fluid chambers 31 and 32; however, the present disclosure is also applicable to a so-called dialysis monitoring apparatus configured to supply a dialysis fluid prepared in advance through the water supply passage 4C.

### Reference Signs List

- 1:: blood purification apparatus
- 2:: dialyzer
- 3:: blood circuit
- 3A:: vein passage
- 3B:: artery passage
- 4:: dialysis fluid circuit
- 4A:: dialysis fluid supply passage
- 4B:: dialysis fluid retrieval passage
- 11:: fluid replenishment circuit
- 12:: priming passage
- 13:: online port

## Claims

1. A blood purification apparatus that includes a blood purifier, an interior of which is divided into a blood flow path and a dialysis fluid flow path by a blood purification membrane, a blood circuit having an artery passage and a vein passage connected to the blood flow path of the blood purifier, a dialysis fluid circuit having a dialysis fluid supply passage and a dialysis fluid retrieval passage connected to the dialysis fluid flow path of the blood purifier, a blood pump provided for the artery passage, a replenishment fluid supply passage connected to the dialysis fluid supply passage, a fluid replenishment passage connected to the blood circuit, an online port which is coupled with the replenishment fluid supply passage and to and from which the fluid replenishment passage can be attached and detached, a water removal passage connected to the dialysis fluid retrieval passage, and a water removal pump provided for the water removal passage, the blood purification apparatus being **characterized in that**:
the online port comprises an inner port connecting the replenishment fluid supply passage to the fluid replenishment passage and an outer port having a tubular shape and being provided around the inner port, and
further provided are a first bypass passage allowing a space formed between the inner port and the outer port to communicate with the dialysis fluid supply passage; and an open/close valve configured to open and close the first bypass passage, wherein
in a fluid draining procedure to eliminate fluid remaining in the blood circuit, by opening the open/close valve on the first bypass passage and discharging fluid from the dialysis fluid retrieval passage by using the water removal pump, air is caused to flow into the first bypass passage through the outer port, and the air is further caused to flow via the dialysis fluid supply passage through the replenishment fluid supply passage toward the blood circuit, so that fluid is thereby removed from the fluid replenishment passage.

2. The blood purification apparatus according to claim 1, **characterized by** comprising:
a second bypass passage allowing the dialysis fluid supply passage to communicate with the dialysis fluid retrieval passage; and
a priming passage being provided branching off from the artery passage and communicating with the second bypass passage, wherein
in the fluid draining procedure, by opening the open/close valve on the first bypass passage and bringing the water removal pump into operation, air is caused to flow into the first bypass passage through the outer port, and the air is caused to flow via the dialysis fluid supply passage and the second bypass passage through the priming passage toward the artery passage, so that fluid is thereby removed from the priming passage.

3. The blood purification apparatus according to claim 1, **characterized in that**:
the blood circuit is provided with a drip chamber, and the drip chamber is provided with an air release valve, wherein
in the fluid draining procedure, by closing the open/close valve on the first bypass passage, opening the air release valve, and bringing the water removal pump and the blood pump into operation, air is caused to flow in through the air release valve, so that the air flows through the blood circuit and pushes out the fluid from the blood flow path into the dialysis fluid flow path at the blood purification membrane of the blood purification apparatus, so that fluid is thereby removed from the blood circuit.
